(19) Europäisches Patentamt
European Patent Office
Office européen des brevets

(11) Publication number: **0 194 047 A1**

(12) **EUROPEAN PATENT APPLICATION**

(21) Application number: **86300886.8**

(22) Date of filing: **10.02.86**

(51) Int. Cl.[4]: **C 07 D 211/90**, C 07 D 401/12, C 07 D 413/04, A 61 K 31/44

(30) Priority: **11.02.85 GB 8503427**

(43) Date of publication of application: **10.09.86 Bulletin 86/37**

(84) Designated Contracting States: **AT BE CH DE FR GB IT LI LU NL SE**

(71) Applicant: **IMPERIAL CHEMICAL INDUSTRIES PLC, Imperial Chemical House Millbank, London SW1P 3JF (GB)**

(72) Inventor: **Hargreaves, Rodney Brian, 42 Deva Close Poyton, Cheshire (GB)**
Inventor: **McLoughlin, Bernard Joseph, 7 Pexhill Drive Macclesfield, Cheshire (GB)**
Inventor: **Mills, Stuart Dennett, 17 Harrington Drive Gawsworth, Macclesfield, SK11 9RD (GB)**
Inventor: **Taylor, Melvin Joseph, 7 Kenmore Road Sale, Cheshire (GB)**

(74) Representative: **Slatcher, Reginald Peter et al, Imperial Chemical Industries PLC Legal Department: Patents P.O. Box 6 Bessemer Road, Welwyn Garden City AL7 1HD (GB)**

(54) **Dihydropyridine alkanol amines, process for their preparation and pharmaceutical compositions containing them.**

(57) A dihydropyridine of the formula:

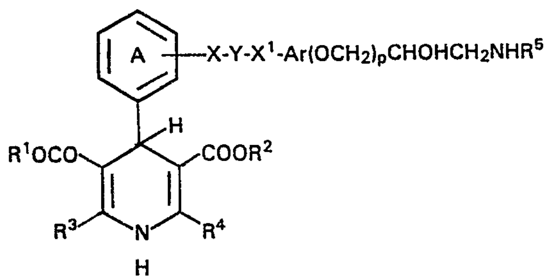

wherein $R^1$ and $R^2$ each is alkyl or alkoxyalkyl, wherein $R^3$ and $R^4$, each is alkyl, wherein $R^5$ is alpha-branched-chain alkyl, alkoxyalkyl, arylalkyl or aryloxyalkyl, or acylaminoalkyl, wherein benzene ring A bears one or more substituents selected from halogeno, cyano, nitro, trifluoromethyl and alkyl, or bears the substituent =N-O-N= attached to the 5- and 6-positions;
wherein Ar is phenylene, naphthylene, tetrahydronaphthylene, indanylene or pyridylene which is unsubstituted or which bears one or more substituents, wherein p is 0 or 1;
wherein X is -O- or -S-;
wherein $X^1$ is a direct link or is -O-, -S-, -NH- or $-NHSO_2-$;
and wherein Y is straight- or branched-chain alkylene which may optionally be interrupted by one or two groups selected from oxygen, sulphur, imino, substituted imino, phenylene, substituted phenylene, pyridylene, cycloalkylene, 1,4-piperazinediyl, 1,4-piperidinediyl and amido groups;
or an acid-addition salt thereof, process for their manufacture and pharmaceutical compositions containing them. The compound possess either beta-adrenergic blocking or calcium ion slow channel blocking properties, or both such properties, and may be used in the treatment of hypertension.

EP 0 194 047 A1

TITLE MODIFIED
see front page

BASIC COMPOUNDS

This invention relates to new basic compounds and more particularly it relates to new dihydropyridine derivatives which possess antihypertensive properties.

Many 2,6-dialkyl-4-aryl-1,4-dihydropyridine-3,5-dicarboxylate derivatives are known which inhibit the movement of calcium ions in the cardiovascular system of warm-blooded animals, and which thereby produce an antihypertensive effect. The most-used of these is nifedipine, which is dimethyl 1,4-dihydro-2,6-dimethyl-4-o-nitrophenylpyridine-3,5-dicarboxylate.

Also known are many 1-aryloxy-3-amino-propan-2-ol derivatives which possess beta-adrenergic receptor blocking properties and which also produce an antihypertensive effect. Two of the most-used of these are propranolol and atenolol, which are respectively 1-(naphth-1-yloxy)- and 1-p-carbamoylmethylphenoxy-3-isopropylaminopropan-2-ol.

The only described attempt to combine these two types of chemical structure into one molecule is reported by Merck workers in the Journal of Medicinal Chemistry, 1981, Vol. 24, pages 628 to 631, in which a 3-amino-2-hydroxypropoxy substituent was introduced into the 4-aryl substituent of a 4-aryl-1,4-dihydropyridine derivative, without much success in producing a compound with antihypertensive activity of the type sought by the authors. More recently, since the priority date of the present application, U.S. Specification No. 4500527 has been published describing similar compounds in which the 3-amino-2-hydroxypropoxy substituent is linked to the 4-aryl substituent by the group -CH=N-, such compounds being claimed to have antihypertensive and beta-adrenergic blocking properties.

We have now found that compounds which do possess useful antihypertensive activity may be obtained by suitably combining a 3-aryloxy-2-hydroxypropylamino moiety with a 1,4-dihydropyridine moiety.

According to the present invention there is provided a dihydropyridine of the formula:

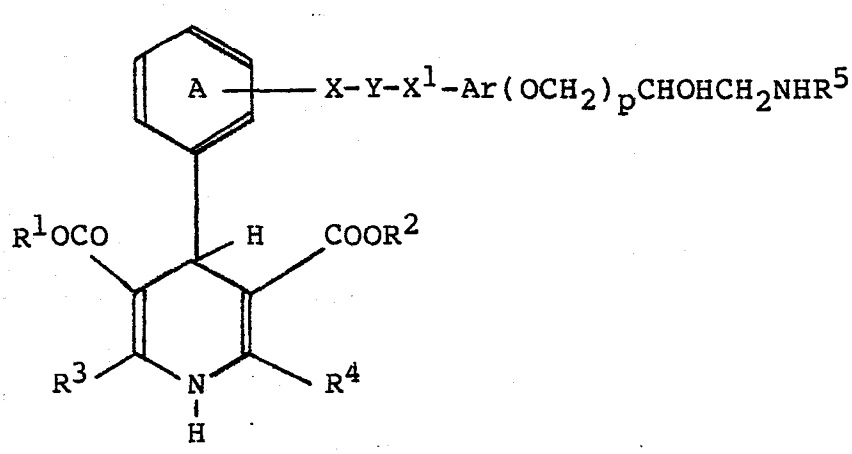

wherein $R^1$ and $R^2$, which may be the same or different, each is alkyl or alkoxyalkyl each of up to 6 carbon atoms:
wherein $R^3$ and $R^4$, which may be the same or different, each is alkyl of up to 6 carbon atoms;
wherein $R^5$ is alpha-branched-chain alkyl or alkoxyalkyl each of up to 6 carbon atoms, or alpha-branched-chain arylalkyl or aryloxyalkyl each of up to 12 carbon atoms or acylaminoalkyl wherein the acyl is of up to 10 carbon atoms and the alkyl is of up to 6 carbon atoms;
wherein benzene ring A bears one or more additional substituents selected from halogeno, cyano, nitro, trifluoromethyl and alkyl of up to 6 carbon atoms or bears the substituent

$$=N-O-N=$$

attached to the 5- and 6-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;
wherein p is 0 or 1;
wherein X is -O- or -S-;
wherein $X^1$ is a direct link or is -O-, -S-, -NH- or $-NHSO_2-$;
and wherein Y is straight-or branched-chain alkylene of 1 to 12 carbon atoms which may optionally be interrupted by one or two groups selected from oxygen (-O-), sulphur (-S-), imino and substituted imino ($-NR^6$ wherein $R^6$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;
or an acid-addition salt thereof.

It will be observed that the dihydropyridine derivative of the invention may possess at least two asymmetric carbon atoms, namely the carbon atom of the -CHOH- group in the alkanolamine chain, and when $R^1$ and $R^2$, or $R^3$ and $R^4$, are different, the carbon atom at the 4-position of the dihydropyridine nucleus, and it can therefore exist in racemic and optically-active forms. It is to be understood that this invention encompasses the racemic form of the dihydropyridine derivative and

any optically-active form which possesses antihypertensive activity, it being a matter of common general knowledge how a racemic compound may be resolved into optically-active forms, and how the antihypertensive activity of these forms may be determined. It is further to be understood that beta-adrenergic blocking activity usually predominates in that optically-active form which has the "S" absolute configuration of the said -CHOH- group in the alkanolamine chain when p is 1 and the "R" absolute configuration when p is 0.

A suitable value for $R^1$, $R^2$, $R^3$, $R^4$, $R^6$ or a substituent in benzene ring A or Ar when it is alkyl is, for example, methyl, ethyl or isopropyl.

A suitable value for $R^1$ or $R^2$ when it is alkoxyalkyl is, for example, methoxyethyl, ethoxyethyl or propoxyethyl.

A suitable value for $R^5$ is, for example, isopropyl, s-butyl, t-butyl, 2-hydroxy-1,1-dimethylethyl, 2-hydroxy-1-methylethyl, 1-methyl-3-phenylpropyl, 1-methyl-2-phenoxyethyl or 2-isobutyramidoethyl.

A suitable halogeno substituent in the benzene ring A or in Ar is, for example fluoro, chloro or bromo.

A suitable value for the alkenyl, alkoxy, alkenyloxy, alkoxyalkoxy, alkylthio, carbamoylalkyl or alkanoylamino substituent in Ar is, for example, allyl, methoxy, ethoxy, isopropoxy, allyloxy, methoxyethoxy, methylthio, carbamoylmethyl or acetamido.

A suitable value for $R^6$ when it is alkanoyl, or for an alkanoyl substituent in Ar is, for example,formyl, acetyl or benzoyl.

A suitable value for $R^6$ when it is aralkyl, or for an aralkyl substituent in Ar is, for example, benzyl.

A suitable value for Y is, for example, straight-chain alkylene of the formula $(-CH_2)_n-$, wherein n is an integer from 1 to 12;

or $-(CH_2)_mC(CH_3)_2-$;

or $-(CH_2)_m-NH-(CH_2)_n-$

$-(CH_2)_m-N(CH_3)-(CH_2)_n-$

$-(CH_2)_m-O-(CH_2)_n-$

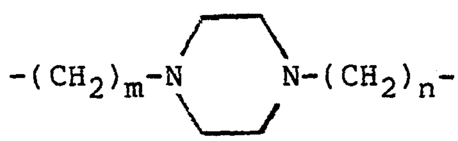

wherein m and n, which may be the same or different, each is 2,3,4 or 5;

or 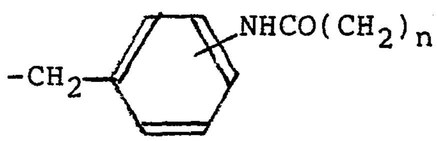 .

$-(CH_2)_mNHCO(CH_2)_n-$

wherein n is 2, 3, 4 or 5 and n is, 1, 2 or 3;

or 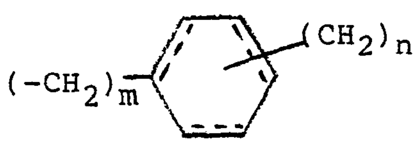 or 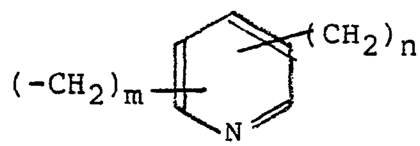

and wherein m and n, which may be the same or different, each is 1, 2, 3 or 4 and wherein the double bonds in the carbocyclic ring are optional (that is, cyclohexylene- or phenylene- bis-alkylene).

A preferred dihydropyridine derivative of the invention is a compound of the formula:-

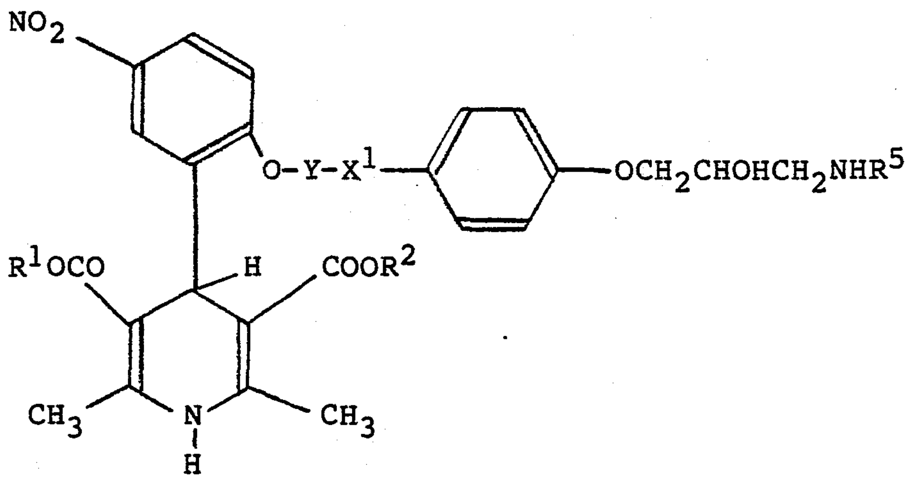

wherein $R^1$ and $R^2$ are either both methyl or both ethyl, wherein $R^5$ is isopropyl or t-butyl and wherein $-Y-X^1-$ is either $-(CH_2)_m-NHCOCH_2-$ or $-(CH_2)_n-O-$ wherein m and n each is 3,4,5 or 6, or an acid-addition salt thereof. A particularly preferred dihydropridine derivative has the last-mentioned formula wherein $R^5$ is isopropyl and m is 3 or 4 or n is 5 or 6.

A suitable acid-addition salt of a dihydropyridine derivative of the invention is, for example, a salt derived from an inorganic acid, for example a hydrochloride, hydrobromide, phosphate or sulphate, or a salt derived from an organic acid, for example an oxalate, lactate, succinate, tartrate, acetate, salicylate, citrate, benzoate, beta-naphthoate or adipate.

Specific dihydropyridine derivatives of the invention are hereinafter described in the Examples. Of these, a preferred compound is dimethyl 4-{2-[4-(2-hydroxy-3-isopropylaminopropoxy)phenylacetamidobutoxy]-5-nitrophenyl -1,4-dihydro-2,6-dimethyl-pyridine-3,5-dicarboxylate.

The dihydropyridine derivatives of the invention may be manufactured by any chemical process known to be useful for the manufacture of chemically-analogous compounds.

One preferred process for the manufacture of a dihydropyridine derivative of the invention comprises the reaction of an epoxide of the formula:-

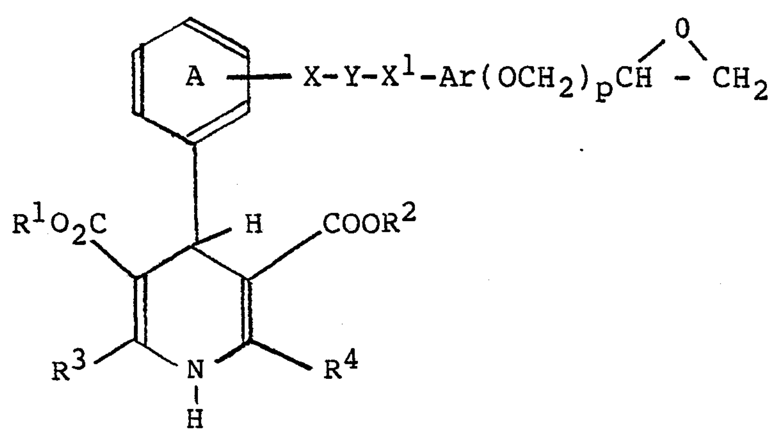

wherein A, $R^1$, $R^2$, $R^3$, $R^4$, X, $X^1$, Y, Ar and p have the meanings stated above, with an amine of the formula:-

$$R^5NH_2$$

wherein $R^5$ has the meanings stated above, or when p is 0, the reaction of said amine with a haloketone of the formula:-

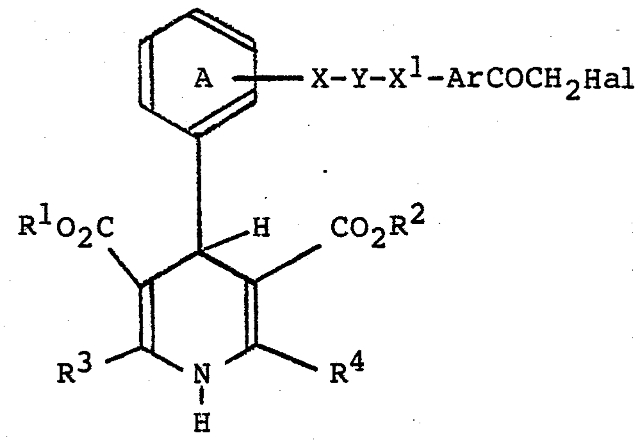

wherein $R^1$, $R^2$, $R^3$, $R^4$, X, $X^1$, Y and Ar have the meanings stated above and wherein Hal stands for a halogen group, for example, bromo, followed by reduction, for example with sodium borohydride, of the aminoketone thus obtained.

The reaction may be carried out in an alcoholic diluent or solvent, for example in isopropanol, at a temperature of up to the boiling point of said diluent or solvent.

A second preferred process for the manufacture of a dihydropyridine derivative of the invention wherein the group -Y- is alkylene interrupted as stated above comprises joining the two parts of the molecule at the point of interruption of Y. Thus, for example, when Y is alkylene interrupted by an amido group -NHCO-, the process comprises the reaction of an amine of the formula:-

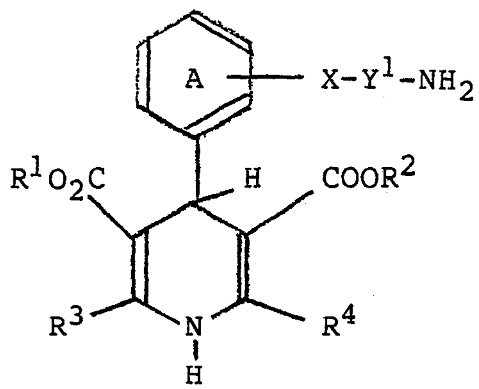

wherein A, $R^1$, $R^2$, $R^3$, $R^4$ and X have the meanings stated above, with an acid of the formula:-

$$HO_2C\text{-}Y^2\text{-}X^1\text{-}Ar(OCH_2)_pCHOHCH_2NHR^5$$

wherein $R^5$, $X^1$, Ar and p have the meanings stated above, or with an activated derivative thereof, and wherein $Y^1$ and $Y^2$ are such that $-Y^1\text{-NHCO-}Y^2-$ has the same meaning as stated above for Y.

A third process for the manufacture of a dihydropyridine derivative of the invention wherein $X^1$ is other than a direct link comprises the reaction of a compound of the formula:-

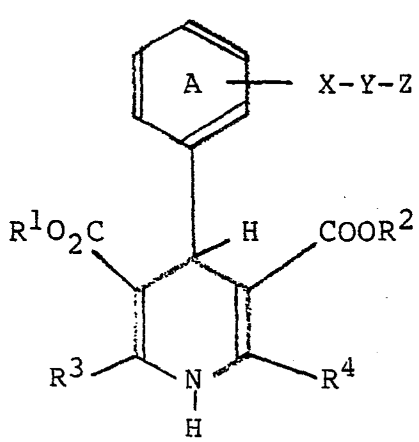

wherein A, $R^1$, $R^2$, $R^3$, $R^4$, X and Y have the meanings stated above and wherein Z stands for a displaceable group, with a compound of the formula:-

$$H\text{-}X^1\text{-}Ar(OCH_2)_pCHOHCH_2NHR^5$$

wherein $R^5$, $X^1$, Ar, and p have the meanings stated above.

A suitable value for Z is, for example, a halogeno group, for example a bromo or chloro group, or a sulphonyloxy group, for example a methanesulphonyloxy or p-toluenesulphonyloxy group.

A fourth process for the manufacture of a dihydropyridine derivative of the invention comprises the reaction of an aldehyde of the formula

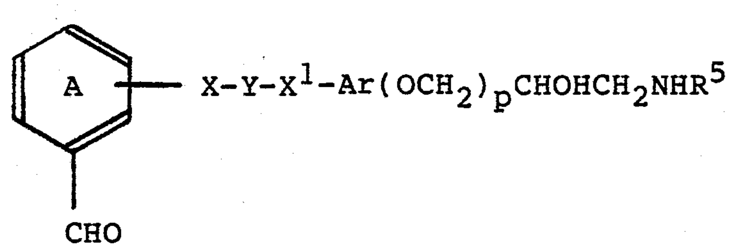

wherein A, X, $X^1$, Y, Ar, p and $R^5$ have the meanings stated above, an aminocrotonate of the formula

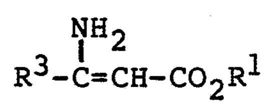

wherein $R^1$ and $R^3$ have the meanings stated above and a ketoacid derivative of the formula

$$R^4COCH_2CO\text{-}OR^2$$

wherein $R^2$ and $R^4$ have the meanings stated above.

This process may be carried out in a diluent or solvent at an elevated temperature, conditions conventionally used for the Hantszch synthesis of dihydropyridines.

As stated above, the dihydropyridine derivatives of the invention possess antihypertensive activity. This may be demonstrated by the ability of the compound to reduce the blood pressure of a spontaneously hypertensive rat, or of a rat made hypertensive by treatment with deoxycorticosterone acetate, or of a dog made hypertensive by the Goldblatt technique of unilateral nephrectomy and clipping of the contralateral kidney. These are all standard tests used to demonstrate antihypertensive effects of medicaments.

Some of the dihydropyridine derivatives of the invention possess beta-adrenergic blocking properties, some of them possess calcium ion slow-channel blocking properties and some of them possess both such activities. A preferred dihydropyridine derivative of the invention possesses both such activities. Beta-adrenergic blocking activity may be demonstrated *in vivo* by the ability of the compound to inhibit isoprenaline-induced tachycardia in a rat or cat, or *in vitro* by shifting to the right the dose- response curve of a guinea pig atrium to isoprenaline. Calcium ion slow channel blocking activity may be demonstrated *in vitro* by the ability of the compound to reduce spontaneous contraction in a rat portal vein preparation. These also are all standard tests used to demonstrate the stated activities.

Because of the beta-adrenergic blocking and/or calcium slow channel blocking properties a dihydropyridine of the invention may also be useful in the

treatment of heart diseases such as angina pectoris and cardiac arrhythmias.

At doses of a dihydropyridine derivative which produce effective antihypertensive activity in a rat or dog no symptom of toxicity is apparent.

The dihydropyridine derivative of the invention may be administered to warm-blooded animals, including man, in the form of a pharmaceutical composition comprising as active ingredient at least one dihydropyridine derivative of the invention, or an acid-addition salt thereof, in association with a pharmaceutically-acceptable diluent or carrier therefor.

A suitable composition is, for example, a tablet, capsule, aqueous or oily solution or suspension, dispersible powder, spray or aerosol formulation.

The pharmaceutical composition may contain, in addition to the dihydropyridine derivative of the invention, one or more drugs selected from sedatives, for example phenobarbitone, meprobamate, chloropromazine and the benzodiazepine sedative drugs, for example chlordiazepoxide and diazepam; vasodilators, for example glyceryl trinitrate, pentaerythritol tetranitrate, isosorbide dinitrate and hydralazine; diuretics, for example chlorthalidone, bendrofluazide, hydrochlorothiazide and chlorothiazide; other antihypertensive agents, for example reserpine, bethanidine and guanethidine; cardiac membrane stabilising agents, for example quinidine; agents used in the treatment of Parkinson's disease and other tremors, for example benzhexol; cardiotonic agents, for example digitalis preparations; and alpha-adrenergic blocking agents, for example phentolamine.

When used for the treatment of heart diseases, for example angina pectoris and cardiac arrhythmias, or

for the treatment of hypertension in man, it is expected that the dihydropyridine derivative would be given to man at a total oral dose of between 20 mg. and 600 mg. daily, or at an intravenous dose of between 1 mg. and 20 mg.

Preferred oral dosage forms are tablets and capsules containing between 10 and 100 mg., and preferably 10 mg. or 50 mg., of active ingredient. Preferred intravenous dosage forms are sterile solutions of the dihydropyridine derivative or of a non- toxic acid-addition salt thereof, containing between 0.05% and 1% w/v of active ingredient, and more particularly containing 0.1% w/v of active ingredient.

The invention is illustrated but not limited by the following Examples:-

Example 1

A mixture of dimethyl 1,4-dihydro-4-[2-(4-p-hydroxyphenylacetamidobutyloxy)-5-nitrophenyl]-2,6-dimethylpyridine-3,5-dicarboxylate (0.56 g.), epichlorohydrin (0.73 g.), methanol (5 ml.) and 25% w/v aqueous sodium hydroxide solution (0.4 ml.) was stirred at laboratory temperature for 18 hours and then evaporated to dryness under reduced pressure. The residue was partitioned between ethyl acetate (30 ml.) and saturated aqueous sodium chloride solution (15 ml.) and the organic phase was separated, washed successively twice with aqueous N-sodium hydroxide solution (15 ml.) each time), aqueous N-hydrochloric acid (15 ml. and saturated aqueous sodium chloride solution (10 ml.), dried over magnesium sulphate and evaporated to dryness. The residual gum was stirred with diethyl ether and the mixture was filtered.

A mixture of the solid dimethyl 4- 2-[4-(p-2,3-epoxypropoxy)phenylacetamidobutyloxy]-5-nitro-

phenyl -1,4-dihydro-2,6-dimethylpyridine-3,5-dicarboxylate thus obtained (0.5 g.), isopropylamine (5 ml.) and isopropanol (15 ml.) was heated under reflux for 8 hours and then evaporated to dryness, and the residue was partitioned between ethyl acetate (30 ml.) and water (20 ml.). The organic phase was separated and extracted twice with aqueous N-hydrochloric acid (20 ml. each time). The combined extracts were basified with cold aqueous 10N-sodium hydroxide solution and extracted twice with ethyl acetate (30 ml. each time). The combined extracts were washed with saturated aqueous sodium chloride solution, dried over magnesium sulphate and evaporated to dryness. The residue was stirred with diethyl ether, the mixture was filtered and the solid product was crystallised from a mixture of ethyl acetate and petroleum ether (b.p. 60-80°C.) and then further purified by flash chromatography on a silica gel column (Merck 9385) using an 80:20:3 v/v/v mixture of ethyl acetate, methanol and concentrated aqueous ammonia solution. The product obtained was stirred with a mixture of diethyl ether and petroleum ether (b.p. 60-80°C.) and the mixture was filtered. There was thus obtained as solid residue dimethyl 1,4-dihydro-4- 2-[4-p(2-hydroxy-3-isopropylaminopropoxy)phenylacetamidobutyloxy]-5-nitrophenyl - 2,6-dimethylpyridine-3,5-dicarboxylate, m.p. 63-67°C. (with decomposition).

The dimethyl pyridinedicarboxylate used as starting material was obtained as follows:-

A stirred mixture of 5-nitrosalicylaldehyde (8.36 g.), potassium carbonate (11.04 g.), potassium iodide (0.2 g.), N-(4-bromobutyl)phthalimide (15.6 g.) and dimethylformamide (120 ml.) was heated at 100°C.for 48 hours, diluted with saturated aqueous sodium chloride solution (1200 ml.) and shaken with ethyl acetate

(300 ml.). The mixture was filtered, the solid product being retained, and the layers of the filtrate were separated. The aqueous layer was extracted with ethyl acetate (200 ml.) and the combined extracts and organic layer were washed three times with saturated aqueous sodium chloride solution (100 ml. each time), dried over magnesium sulphate and evaporated to dryness. The residue and the retained solid product were crystallised from methanol and there was thus obtained 5-nitro-2-(4-phthalimidobutyloxy)benzaldehyde, m.p. 145-148°C.

A stirred mixture of the above aldehyde (5.68 g.), methyl 3-aminocrotonate (1.92 g.), methyl acetoacetate (1.79 g.) and isopropanol (30 ml.) was heated under reflux for 40 hours, cooled and the liquid was decanted from the precipitated gum. The liquid was kept at laboratory temperature for 18 hours and then filtered, and the precipitated gum was stirred with diethyl ether and the mixture was filtered. The combined solid residues were crystallised from isopropanol and there was thus obtained dimethyl 1,4-dihydro-2,6-dimethyl-4-[2-(4-phthalimidobutyloxy)-5-nitrophenyl]pyridine-3,5-dicarboxylate, m.p. 119-123°C.

A mixture of the above compound (1.8 g.), hydrazine hydrate (1.8 ml.) and ethanol (35 ml.) was heated under reflux for 18 hours, cooled, kept at laboratory temperature for 18 hours and filtered. The filtrate was evaporated to dryness and the residue was crystallised from ethanol. There was thus obtained dimethyl 4-[2-(4-aminobutyloxy)-5-nitrophenyl]-1,4-dihydro-2,6-dimethylpyridine-3,5-dicarboxylate, m.p. 200-202°C.

A mixture of the above compound (0.5 g.), p-hydroxyphenylacetic acid (0.18 g.), 1-hydroxybenzotriazole (0.17 g.), dicyclohexylcarbodiimide (0.29 g.) and methylene chloride (20 ml.) was stirred at

laboratory temperture for 24 hours, filtered and the filtrate was washed successively twice with aqueous N-hydrochloric acid (20 ml. each time), twice with saturated aqueous sodium bicarbonate solution (20 ml.each time) and saturated aqueous sodium chloride solution (20 ml.), filtered through phase-separating paper and evaporated to dryness. The residue was stirred with cold ethyl acetate, the mixture was filtered and the filtrate was evaporated to dryness. There was thus obtained as residue the desired dimethyl 1,4-dihydro-4-[2-(4-p-hydroxyphenylacetamidobutyloxy)-5-nitrophenyl]-2,6-dimethylpyridine-3,5-dicarboxylate which was used without further purification.

Example 2

The process described in Example 1 was repeated using diethyl 1,4-dihydro-4-[2-(3-p-hydroxyphenylacetamidopropoxy)-5-nitrophenyl]-2,6-dimethylpyridine-3,5-dicarboxylate as starting material. There was thus obtained as an oil diethyl 1,4-dihydro-4-{2-[3-p-(2-hydroxy-3-isopropylaminopropoxy)phenyl-acetamidopropoxy]-5-nitrophenyl}-2,6-dimethylpyridine-3,5-dicarboxylate, the structure of which was confirmed by elemental analysis and proton magnetic resonance spectroscopy.

The starting material was prepared by a similar method to that described in Example 1 except that N-(3-bromopropyl)phthalimide was used in place of N-(4-bromobutyl)phthalimide, and ethyl 3-aminocrotonate was used in place of methyl 3-aminocrotonate. The intermediate diethyl 4-[2-(3-aminopropoxy)-5-nitrophenyl]-1,4-dihydro-2,6-dimethylpyridine-3,5-dicarboxylate had m.p. 187-190°C. after crystallisation from ethanol.

Example 3

The process described in Example 1 was repeated using dimethyl 1,4-dihydro-4-[2-(5-p-hydroxyphenoxypentyloxy)-5-nitrophenyl]-2,6-dimethylpyridine-3,5-dicarboxylate or the corresponding 6-p-hydroxyphenoxyhexyloxy compound as starting material. There were thus obtained as oils, respectively, dimethyl 1,4-dihydro-4-{2-[5-p-(2-hydroxy-3-isopropylaminopropoxy)phenoxypentyloxy]-5-nitrophenyl}-2,6-dimethylpyridine-3,5-dicarboxylate and dimethyl 1,4-dihydro-4-{2-[6-p-(2-hydroxy-3-isopropylaminopropoxy)phenoxyhexyloxy]-5-nitrophenyl}-2,6-dimethylpyridine-3,5-dicarboxylate, the structures of both of which were confirmed by elemental analysis and proton magnetic resonance spectroscopy.

The starting materials were prepared by a similar method to that described in the third paragraph of Example 1 using p-5-bromopentyloxyphenol or p-6-bromohexyloxyphenol in place of N-(4-bromobutyl)phthalimide. 2-(5-p-Hydroxyphenoxypentyloxy)-5-nitrobenzaldehyde has m.p. 65-70°C. and 2-(6-p-hydroxyphenoxyhexyloxy)-5-nitrobenzaldehyde has m.p. 100-102°C. after crystallisation from isopropanol.

The above aldehydes were then reacted with methyl 3-aminocrotonate and methyl acetoacetate in methanol solution by a similar process to that described in the fourth paragraph of Example 1. Dimethyl 1,4-dihydro-4-[2-(5-p-hydroxyphenoxypentyloxy)-5-nitrophenyl]-2,6-dimethylpyridine-3,5-dicarboxylate has m.p. 197-198°C. after crystallisation from ethanol, and dimethyl 1,4-dihydro-4-[2-(6-p-hydroxyphenoxyhexyloxy)-5-nitrophenyl]-2,6-dimethylpyridine-3,5-dicarboxylate has m.p. 188-192°C. after crystallisation from isopropanol.

What we claim is:

1. A dihydropyridine of the formula:-

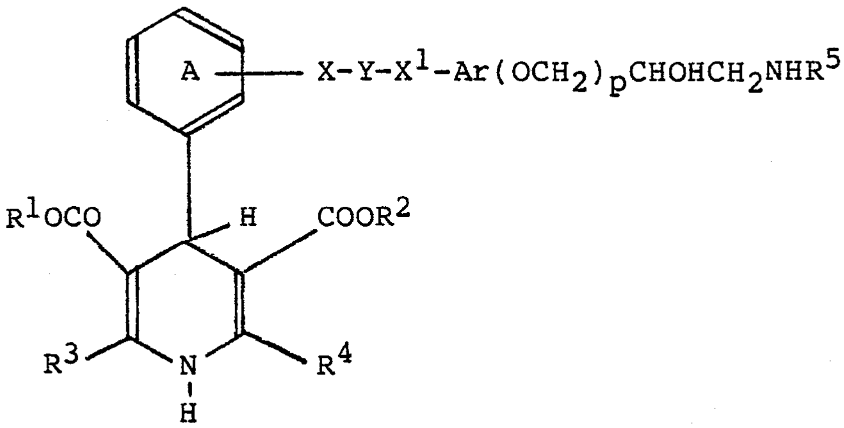

wherein $R^1$ and $R^2$, which may be the same or different, each is alkyl or alkoxyalkyl each of up to 6 carbon atoms:
wherein $R^3$ and $R^4$, which may be the same or different, each is alkyl of up to 6 carbon atoms;
wherein $R^5$ is alpha-branched-chain alkyl or alkoxyalkyl each of up to 6 carbon atoms, or alpha-branched-chain arylalkyl or aryloxyalkyl each of up to 12 carbon atoms or acylaminoalkyl wherein the acyl is of up to 10 carbon atoms and the alkyl is of up to 6 carbon atoms;
wherein benzene ring A bears one or more additional substituents selected from halogeno, cyano, nitro, trifluoromethyl and alkyl of up to 6 carbon atoms or bears the substituent

$$=N-O-N=$$

attached to the 5- and 6-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents

selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;

wherein p is 0 or 1;

wherein X is -O- or -S-;

wherein $X^1$ is a direct link or is -O-, -S-, -NH- or $-NHSO_2-$;

and wherein Y is straight-or branched-chain alkylene of 1 to 12 carbon atoms which may optionally be interrupted by one or two groups selected from oxygen (-O-), sulphur (-S-), imino and substituted imino ($-NR^6$ wherein $R^6$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;

or an acid-addition salt thereof.

2. A dihydropyridine as claimed in claim 1 which has the formula:-

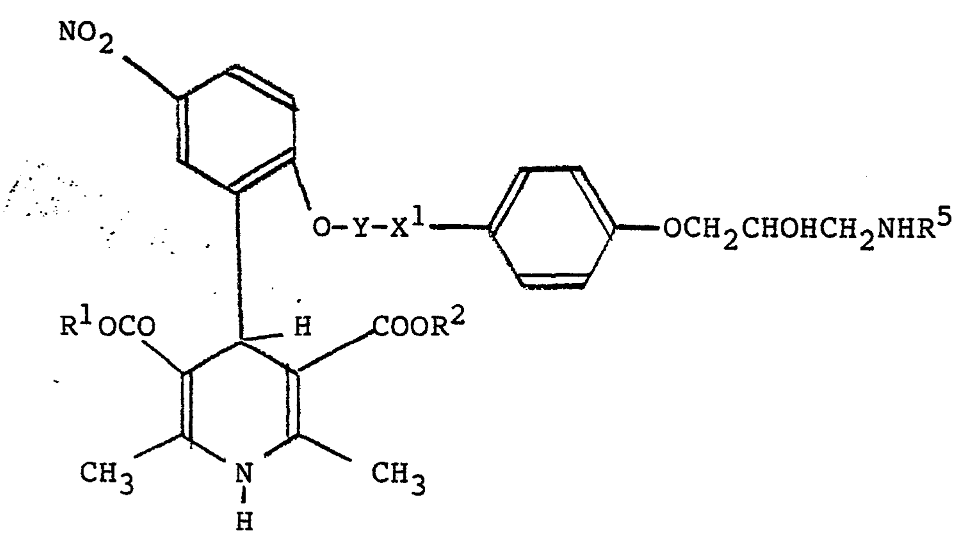

wherein $R^1$ and $R^2$ are either both methyl or both ethyl, wherein $R^5$ is isopropyl or t-butyl and wherein $-Y-X^1-$ is either $-(CH_2)_m-NHCOCH_2-$ or $-(CH_2)_n-O-$ wherein m and n each is 3,4,5 or 6, or an acid-addition salt thereof.

3. A dihydropyridine as claimed in claim 2 wherein $R^5$ is isopropyl and m is 3 or 4 or n is 5 or 6.

4. The compound dimethyl 4-{2-[4-(2-hydroxy-3-isopropylaminopropoxy)phenylacetamidobutoxy]-5-nitrophenyl}-1,4-dihydro-2,6-dimethyl-pyridine-3,5-dicarboxylate.

5. A process for the manufacture of a dihydropyridine, claimed in any of claims 1 to 4,which comprises

(a) the reaction of an epoxide of the formula:-

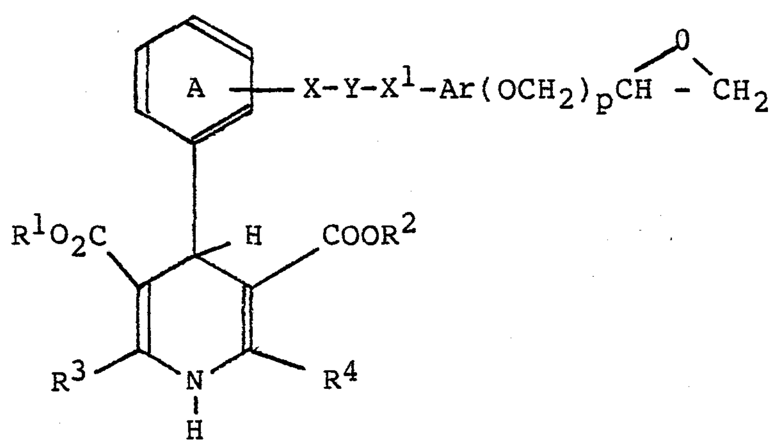

wherein A, $R^1$, $R^2$, $R^3$, $R^4$, X, $X^1$, Y, Ar and p have the meanings stated in any of claims 1 to 3, with an amine of the formula:-

$$R^5NH_2$$

wherein $R^5$ has the meanings stated in any of claims 1 to 3, or when p is 0, the reaction of said amine with a haloketone of the formula:-

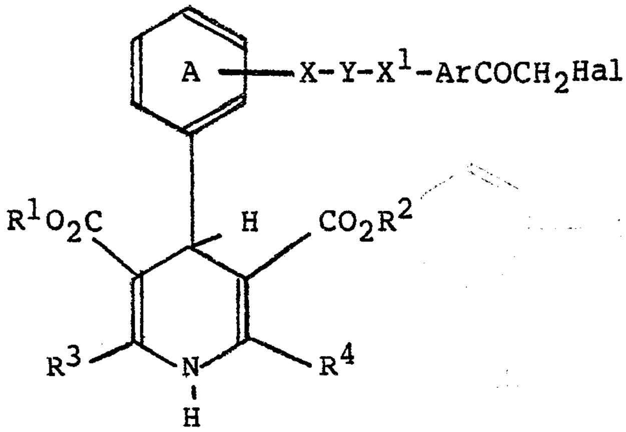

wherein $R^1$, $R^2$, $R^3$, $R^4$, X, $X^1$, Y and Ar have the meanings stated above and wherein Hal stands for a halogen group, followed by reduction of the aminoketone thus obtained; or

(b) for the manufacture of a dihydropyridine wherein the group -Y- is alkylene interrupted by an amido group -NHCO-, the reaction of an amine of the formula:-

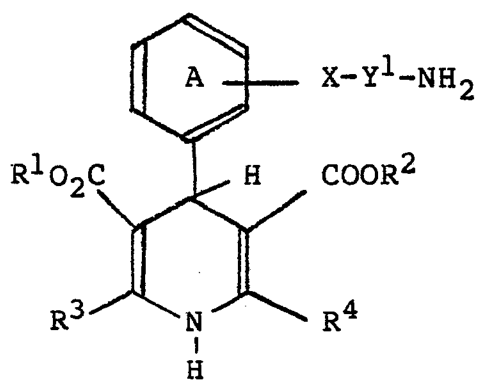

wherein A, $R^1$, $R^2$, $R^3$, $R^4$ and X have the meanings stated above, with an acid of the formula:-

$$HO_2C\text{-}Y^2\text{-}X^1\text{-}Ar(OCH_2)_pCHOHCH_2NHR^5$$

wherein $R^5$, $X^1$, Ar and p have the meanings stated above, or with an activated derivative thereof, and wherein $Y^1$ and $Y^2$ are such that $-Y^1\text{-}NHCO\text{-}Y^2-$ has the same meaning as stated above for Y; or

(c) for the manufacture of a dihydropyridine wherein $X^1$ is other than a direct link, the reaction of a compound of the formula:-

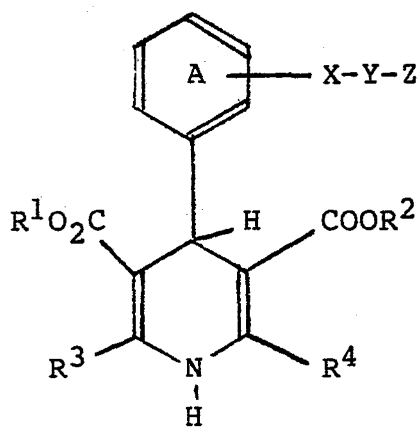

wherein A, $R^1$, $R^2$, $R^3$, $R^4$, X and Y have the meanings stated above and wherein Z stands for a displaceable group, with a compound of the formula:-

$$H\text{-}X^1\text{-}Ar(OCH_2)_pCHOHCH_2NHR^5$$

wherein $R^5$, $X^1$, Ar, and p have the meanings stated above; or

(d) the reaction of an aldehyde of the formula

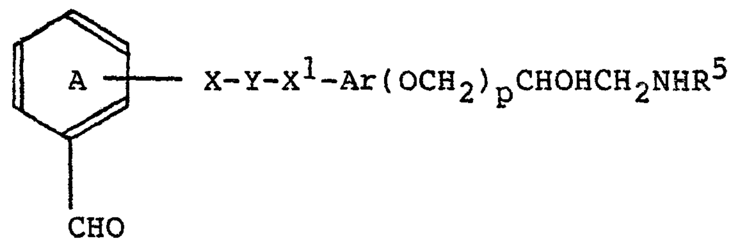

wherein A, X, $X^1$, Y, Ar, p and $R^5$ have the meanings stated above, an aminocrotonate of the formula

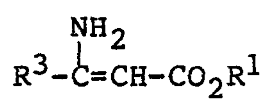

wherein $R^1$ and $R^3$ have the meanings stated above and a ketoacid derivative of the formula

$$R^4COCH_2CO\text{-}OR^2$$

wherein $R^2$ and $R^4$ have the meanings stated above.

6. A pharmaceutical composition comprising as active ingredient at least one dihydropyridine, claimed in any of claims 1 to 4, or an acid-addition salt thereof, in association with a pharmaceutically-acceptable diluent or carrier therefor.

7. A composition as claimed in claim 6 which is a tablet, capsule, aqueous or oily solution or suspension, dispersible powder, spray or aerosol formulation.

8. A composition as claimed in claim 6 or 7 which contains, in addition to the dihydropyridine, one or more drugs selected from sedatives, vasodilators, diuretics, other antihypertensive agents, cardiac membrane stabilising agents, agents used in the treatment of Parkinson's disease and other tremors,

cardiotonic agents, and alpha-adrenergic blocking agents.

9. The use of a compound, claimed in any of claims 1 to 4, for the manufacture of a medicament for producing an antihypertensive effect in a warm-blooded animal.

10. A method for the treatment of heart disease or hypertension in a warm-blooded animal which comprises administering to said animal an effective amount of a compound claimed in claim 1.

REGINALD PETER SLATCHER

AUTHORISED REPRESENTATIVE
General Authorisation No. 97

SE01
PS33367
RPS/MEL: 08 JAN 86

CLAIMS FOR AUSTRIA

What we claim is:

1. A process for the manufacture of a dihydropyridine of the formula:-

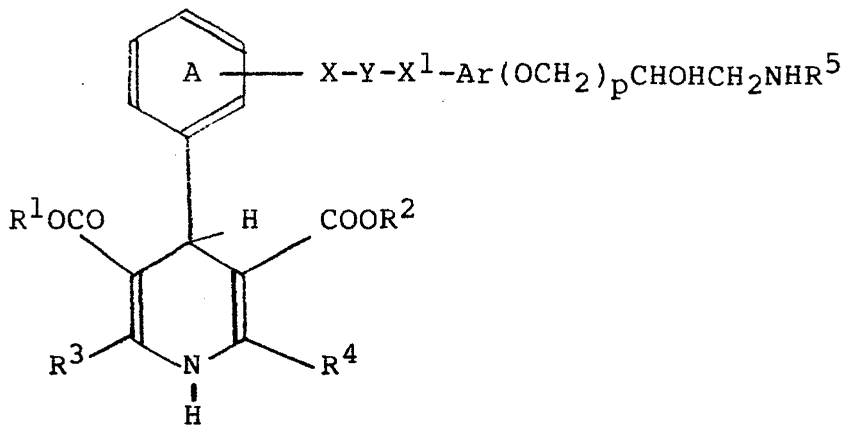

wherein $R^1$ and $R^2$, which may be the same or different, each is alkyl or alkoxyalkyl each of up to 6 carbon atoms:
wherein $R^3$ and $R^4$, which may be the same or different, each is alkyl of up to 6 carbon atoms;
wherein $R^5$ is alpha-branched-chain alkyl or alkoxyalkyl each of up to 6 carbon atoms, or alpha-branched-chain arylalkyl or aryloxyalkyl each of up to 12 carbon atoms or acylaminoalkyl wherein the acyl is of up to 10 carbon atoms and the alkyl is of up to 6 carbon atoms;
wherein benzene ring A bears one or more additional substituents selected from halogeno, cyano, nitro, trifluoromethyl and alkyl of up to 6 carbon atoms or bears the substituent

=N-O-N=

attached to the 5- and 6-positions (that is, to form a benzo-2,1,3-oxadiazole nucleus);
wherein Ar is phenylene, naphthylene, tetrahydronaphthylene, indanylene or pyridylene which is unsubstituted or which bears one or more substituents

selected from halogeno, trifluoromethyl, hydroxy, amino, nitro, carbamoyl and cyano, and alkyl, alkenyl, alkoxy, alkenyloxy, alkoxyalkoxy, alkylthio, alkanoyl, carbamoylalkyl and alkanoylamino each of up to 6 carbon atoms;

wherein p is 0 or 1;

wherein X is -O- or -S-;

wherein $X^1$ is a direct link or is -O-, -S-, -NH- or $-NHSO_2-$;

and wherein Y is straight-or branched-chain alkylene of 1 to 12 carbon atoms which may optionally be interrupted by one or two groups selected from oxygen (-O-), sulphur (-S-), imino and substituted imino ($-NR^6$ wherein $R^6$ is hydrogen, alkyl or alkanoyl each of up to 10 carbon atoms, phenyl or aralkyl of up to 12 carbon atoms), phenylene, substituted phenylene, pyridylene, cycloalkylene of up to 6 carbon atoms, 1,4-piperazinediyl, 1,4-piperidinediyl and amido (-CONH- or -NHCO-) groups;

or an acid-addition salt thereof, characterised by:-

(a) the reaction of an epoxide of the formula:-

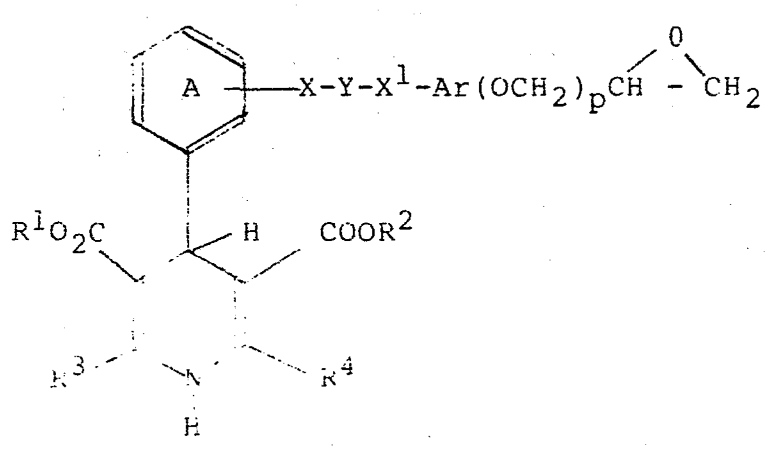

wherein A, $R^1$, $R^2$, $R^3$, $R^4$, X, $X^1$, Y, Ar and p have the meanings stated above, with an amine of the formula:-

BAD ORIGINAL

$R^5NH_2$

wherein $R^5$ has the meanings stated above or, when p is 0, the reaction of said amine with a haloketone of the formula:-

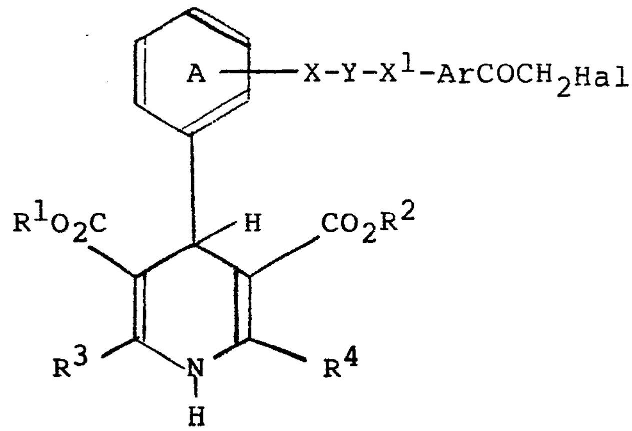

wherein $R^1$, $R^2$, $R^3$, $R^4$, X, $X^1$, Y and Ar have the meanings stated above and wherein Hal stands for a halogen group, followed by reduction of the aminoketone thus obtained; or

(b) for the manufacture of a dihydropyridine wherein the group -Y- is alkylene interrupted by an amido group -NHCO-, the reaction of an amine of the formula:-

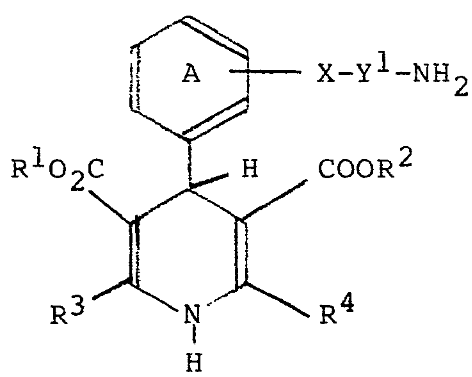

wherein A, $R^1$, $R^2$, $R^3$, $R^4$ and X have the meanings stated above, with an acid of the formula:-

$$HO_2C-Y^2-X^1-Ar(OCH_2)_pCHOHCH_2NHR^5$$

wherein $R^5$, $X^1$, Ar and p have the meanings stated above, or with an activated derivative thereof, and wherein $Y^1$ and $Y^2$ are such that $-Y^1-NHCO-Y^2-$ has the same meaning as stated above for Y; or

(c) for the manufacture of a dihydropyridine wherein $X^1$ is other than a direct link, the reaction of a compound of the formula:-

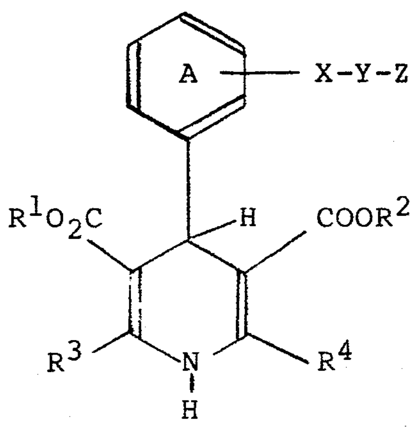

wherein A, $R^1$, $R^2$, $R^3$, $R^4$, X and Y have the meanings stated above and wherein Z stands for a displaceable group, with a compound of the formula:-

$$H-X^1-Ar(OCH_2)_pCHOHCH_2NHR^5$$

wherein $R^5$, $X^1$, Ar, and p have the meanings stated above; or

(d) the reaction of an aldehyde of the formula

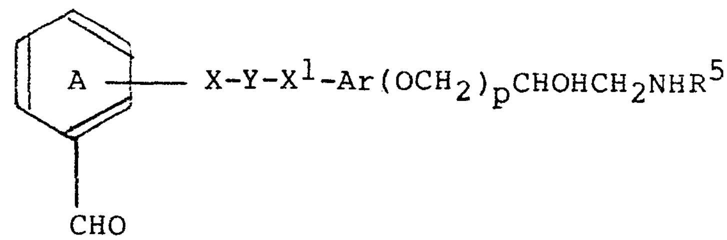

wherein A, X, $X^1$, Y, Ar, p and $R^5$ have the meanings stated above, an aminocrotonate of the formula

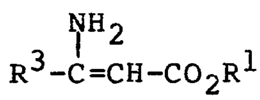

wherein $R^1$ and $R^3$ have the meanings stated above and a ketoacid derivative of the formula

$$R^4COCH_2CO\text{-}OR^2$$

wherein $R^2$ and $R^4$ have the meanings stated above.

2. A process as claimed in claim 1 for the manufacture of a compound of the formula:-

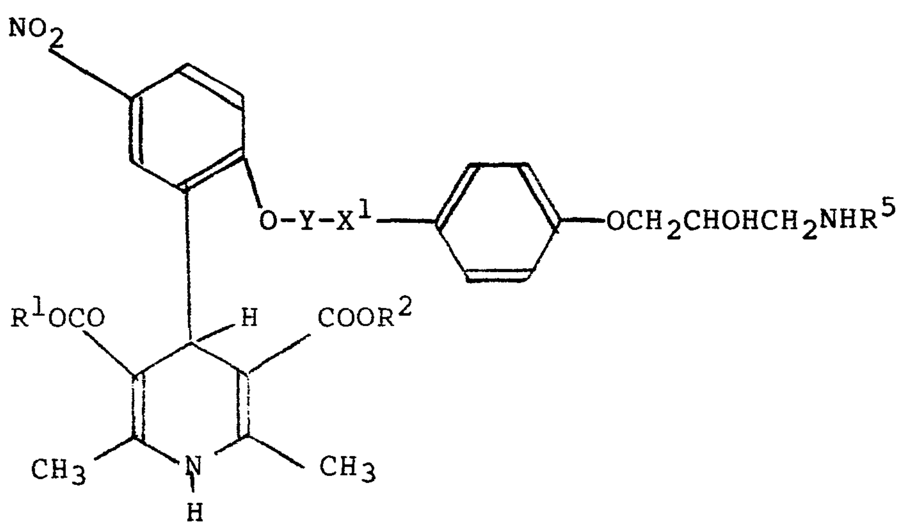

wherein $R^1$ and $R^2$ are either both methy or both ethyl, wherein $R^5$ is isopropyl or t-butyl and wherein $-Y-X^1$ is either $-(CH_2)_m-NHCOCH_2-$ or $-(CH_2)_n-O-$ wherein m and n each is 3,4,5 or 6, or an acid-addition salt thereof.

3. A process as claimed in claim 2 wherein $R^5$ is isopropyl and m is 3 or 4 or n is 5 or 6.

REGINALD PETER SLATCHER

AUTHORISED REPRESENTATIVE
General Authorisation No. 97

PS33367AT
SE01
RPS/MJW: 6 Feb 86

0194047

European Patent Office

# EUROPEAN SEARCH REPORT

Application number

EP 86 30 0886

| Category | Citation of document with indication, where appropriate, of relevant passages | Relevant to claim | CLASSIFICATION OF THE APPLICATION (Int. Cl.4) |
|---|---|---|---|
| A | GB-A-1 409 865 (SCIENCE UNION) * Example 10; pages 6,7; claims * --- | 1,5-10 | C 07 D 211/90 C 07 D 401/12 C 07 D 413/04 A 61 K 31/44 |
| A | GB-A-1 425 059 (BAYER) * Examples 6,7; page 15; claims * --- | 1,5-10 | |
| D,A | JOURNAL OF MEDICINAL CHEMISTRY, vol. 24, no. 5, May 1981, pages 628-631, American Chemical Society, Washington, US; J.J. BALDWIN et al.: "Approaches to vasodilating/bêta-adrenergic blocking agents: examples of the dihydrolutidine type" * Page 629, cmpounds 4a,b,c; page 631, experimental section; pages 630-631, discussion * --- | 1,5-10 | |
| | | | TECHNICAL FIELDS SEARCHED (Int. Cl.4) |
| D,P A | US-A-4 500 527 (B. LOEV) * Whole document * --- | 1,5-10 | C 07 D 21 C 07 D 401 C 07 D 413 A 61 K 31 |
| P,A | EP-A-0 167 371 (YAMANOUCHI PHARMACEUTICAL CO., LTD.) * Whole document, especially examples 1-46 * ----- | 1,5-10 | |

The present search report has been drawn up for all claims

| Place of search | Date of completion of the search | Examiner |
|---|---|---|
| THE HAGUE | 26-05-1986 | NUYTS A.M.K.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document